# EUROPEAN PATENT APPLICATION

(11) **EP 2 518 500 A1**
(43) Date of publication of application: **31.10.2012**
(21) Application number: 11163981.1
(22) Date of filing: 27.04.2011
(51) Int. Cl.: G01N 33/50

(54) **In vitro method for determining developmental toxicity of a compound.**

(71) Applicant: Universiteit Maastricht, 6211 LK Maastricht (NL); Academisch Ziekenhuis Maastricht, 6229 HX Maastricht (NL); Rijksinstituut Voor Volksgezondheid En Milieu, 3721 MA Bilthoven (NL)
(72) Inventor: Pennings, Jeroen Lambertus Antonius, 3562 TM Utrecht (NL); Hermsen, Sanne Anna Bertina, 5341 AS Oss (NL); Piersma, Aldert Henrick, 3720 BA Bilthoven (NL); Pronk, Theresia Elizabeth, 3994 XR Houten (NL)
(74) Representative: Habets, Winand

(57) **Abstract**

The invention is in the field of medical molecular diagnostics. It provides methods and means for the *in vitro* detection of the developmental toxicity of a compound by determining the gene expression of a limited number of genes. More in particular, it provides a method for determining the developmental toxicity of a compound comprising the steps of providing a 2 - 128 cell stage fish embryo, contacting the fish embryo with the compound, allowing the embryo to develop for an amount of time in the presence of the compound, isolating total RNA from the embryo, determining the expression level of at least one gene selected from the group consisting of *cyp26a1, si:rp71-39b20.7, vmhc* and *hspb12,* comparing the expression level of said at least one gene with a predetermined reference value, wherein an increase or decrease of the expression level of said gene indicates that the compound is developmentally toxic.

## Description

### Field of the invention

The invention is in the field of medical molecular diagnostics. It provides methods and means for the in vitro detection of the developmental toxicity of a compound by determining the gene expression of a limited number of genes.

### Background of the invention

A wide variety of assays for developmental toxicity testing have been developed over the years, varying from cell-line based tests to different organ cultures and whole embryo cultures of rat or zebrafish [1-4].

The zebrafish embryotoxicity test (ZET) is a test system which uniquely enables the study of effects of chemicals on the entire developmental period of a complete vertebrate embryo in a simple and fast culture system [5-8]. Advantages of the ZET include the detailed knowledge available about embryogenesis in the zebrafish and the independence of embryo development from the maternal fish. In addition, the transparency of the embryos makes them easy to monitor and evaluate. Furthermore, development takes only three days from fertilization until hatching and at these stages the zebrafish embryo is not considered a laboratory animal under European legislation [9]. These advantages make the zebrafish embryo model suitable for relatively high-throughput tests.

Most of the test systems that use zebrafish embryos rely on the morphological assessment of effects in the embryos after exposure to compounds [2, 10]. We recently published a standardized morphological scoring system and evaluated the developmentally toxic effects of compounds after 72 hours post fertilization (hpf) [11]. However, subtle responses or effects becoming visible only at later time points may remain unnoticed by morphological scoring.

There is a need in the art for more reliable, sensitive and specific assays for determining the developmental toxicity of a compound.

### Summary of the invention

We found that the embryotoxicity of a compound may be assessed by determining the expression level of any of 4 genes in a developing fish embryo.

Hence, the invention relates to a method for determining the developmental toxicity of a compound comprising the steps of
a) Providing a 2 - 128 cell stage fish embryo,
b) Contacting the fish embryo with the compound,
c) Allowing the embryo to develop for an amount of time in the presence of the compound,
d) Isolating total RNA from the embryo,
e) Determining the expression level of at least one gene selected from the group consisting of *cyp26a1, si:rp71-39b20.7, vmhc* and *hspb12,*
f) Comparing the expression level of said at least one gene with a predetermined reference value,
wherein an increase or decrease of the expression level of said gene of 50% indicates that the compound is developmentally toxic.

### Detailed description of the invention

It was found that the developmental toxicity of a compound could be determined by analyzing the expression level of at least one out of 4 distinct genes in a fish embryo contacted with the compound.

In one aspect, the invention therefore relates to a method for determining the developmental toxicity of a compound comprising the steps of
a) Providing a 2 - 128 cell stage fish embryo,
b) Contacting the fish embryo with the compound,
c) Allowing the embryo to develop for an amount of time in the presence of the compound,
d) Isolating total RNA from the embryo,
e) Determining the expression level of at least one gene selected from the group consisting of *cyp26a1, si:rp71-39b20.7, vmhc* and *hspb12,*
f) Comparing the expression level of said at least one gene with a predetermined reference value,
wherein an increase or decrease of the expression level of said gene indicates that the compound is developmentally toxic.

As used herein, the terms "developmentally toxic", "developmental toxicity" are equivalent and intended to refer to the property of a compound to interfere with the development of an embryo.

We found that the gene expression of 4 genes was altered when exposed to four different developmentally toxic compounds. When the expression level of these 4 genes was compared to a reference value, it was found that *vmhc*, *si:rp71-39b20.7* and *hspb12* were downregulated and *cyp26a1* was upregulated (Table 1).

Fish embryos in a 2 - 128 cell stadium were exposed to the compounds and tested for gene expression. For these purposes fish embryos are preferred since they are easy to culture at low costs and are not considered laboratory animals under European legislation in the stages used in this test [9]. The method according to the invention may however be employed with other embryos without deviating from the inventive concept thereof. In a preferred embodiment, the fish is a zebrafish, however other fish species may also be used, for instance medaka or fathead minnow.

The fish embryos were contacted with the compounds in solution. It should be noted that only sub-toxic concentrations of the compounds should be used. Such sub-toxic concentrations can easily be determined by a skilled person using his routine skills, for instance by incubating the embryos with limiting dilutions of the compound in culture solution.

Next, the embryo is allowed to develop in the presence of the compound for a certain period of time. For each individual system, the optimal exposure time may be determined, in our experimental setting, a time between 12 and 72 hours was found most convenient. Preferably, the exposure time is between 20 and 28 hours, such as 21, 22, 23 24, 25, 26 or 27 hours.

After the exposure, total RNA is isolated from the embryo. This may be performed in any way known in the art, for instance by using a standardized kit such as the RNeasy mini kit from Qiagen or using the method as described by De Jong *et al.* [12]. With both methods RNA of good quality can be obtained.

Total RNA may be used to determine the expression levels of any of the 4 genes disclosed herein. This may be done preferably by quantitative real-time PCR or microarray analysis, given costs and work effort required under current state of the art; but it can also be done by or other methods such as Northern blot analysis, Whole Transcriptome Shotgun Sequencing (RNA-seq), or serial analysis of gene expression (SAGE).

Reference values for the expression levels of the genes when not exposed to a developmentally toxic compound may be obtained by performing a method as described above without contacting the embryo with a compound. Reference values may also be obtained by performing the method according to the invention with a known non-developmental toxic compound. It is preferred to use a reference value obtained without contacting the embryo with a compound.

In a preferred embodiment, the increased or decreased expression level of the genes identified herein is at least 50% of the reference value. This is a difference that can easily be detected by the above mentioned methods for determining gene expression and therefore allows for robust measurement.

In another preferred embodiment, the method according to the invention is performed wherein the fish embryo is provided in a 4 - 64 cell stage.

The results obtained by the method were found to be more accurate when the subsequent method steps a) to f) were repeated and wherein the increase or decrease of said gene expression was calculated as an average of the independent repeats. The invention therefore relates to a method as described above, wherein the subsequent method steps a) to f) are repeated and wherein the increase or decrease of said gene expression is an average of the independent repeats.

The compounds used for the examples as described herein belong to the classes of established developmentally toxic compounds known as glycol ethers and triazoles. These compounds were classified as developmentally toxic in the method according to the invention whereas the compound PAA was found non-developmentally toxic.

Glycol ethers are mostly used as solvents in paints and inks. Some of them exhibit embryotoxic properties in rat, mouse, rabbit and zebrafish [11, 13-15], including visceral and skeletal malformations. The mechanism of action for methoxyacetic acid (MAA), one of the developmentally toxic glycol ether metabolites, is thought to be related to the interference with nuclear-receptor mediated signaling [16, 17].

Triazoles are used as fungicides and some of them also have the potential to affect development in different species, such as rat and mouse [18-20]. One proposed pathway by which these compounds may cause their effect is the altered expression of the cytochrome P450 isoenzyme CYP26 [21-24]. By affecting the endogenous retinoic acid levels in the embryo this can ultimately lead to craniofacial and axial skeletal malformations [25-27].

Especially, *cyp26a1,* is interesting for the prediction of developmental toxicity, since this gene is strongly upregulated by both classes of compounds. Furthermore, in morphological assays such as the zebrafish embryotoxicity test, the effects of triazoles can only be detected after 72 hours post fertilization. The method according to the invention scored these compounds as developmentally toxic after 24 hours of fertilization.

When the expression levels of more than one gene, such as 2, 3 or 4 genes selected from the group consisting of genes *cyp26a1*, *si:rp71-39b20.7*, *vmhc* and *hspb12,* are determined and used in the method according to the invention, the quality of the method may even be improved. Quality in this respect is to be measured as accuracy, sensitivity, specificity or positive and negative predicted value.

**Table 1**

| | | | Fold ratio (FR) | | | | |
|---|---|---|---|---|---|---|---|
| GenelD | Gene | Official gene name | PAA | MAA | EAA | FLU | CYP |
| 30381 | *cyp26a1* | cytochrome P450, subfamily XXVIA, polypeptide 1 | -1.009 | 1.722 | 2.648 | 3.452 | 3.745 |
| 492647 | *si:rp71-39b20.7* | si:rp71-39b20.7 | -1.098 | -1.996 | -2.183 | -1.514 | -1.544 |
| 30616 | *vmhc* | ventricular myosin heavy chain | -1.102 | -2.946 | -4.629 | -1.784 | -1.759 |
| 324021 | *hspb12* | heat shock protein, alpha-crystallin-related, b12 | -1.135 | -3.449 | -16.913 | -2.182 | -1.531 |

Expression values (FR) of 1 equals unchanged compared to the control. Negative numbers indicate downregulation compared to the control, positive numbers indicate upregulation. The FR is calculated comparing the normalized signal of the compound-exposed samples relative to the average normalized signal of the DMSO-exposed samples (control). For downregulated samples this value was converted to the inverse negative FR. If for instance the compound-control FR is 0.5, this was converted to -2, since the expression value of the compound was 2-fold downregulated compared to the control.

Table 1 shows the results obtained as described in the examples section.

### Examples

### Example 1:Maintenance and breeding of zebrafish

Danio rerio used were originally obtained as commercially imported Singapore wild type stock, maintained and bred in our facilities for several years.

### Example 2: Zebrafish embryotoxicity test

The ZET was performed as previously described [11]. In short, fertilized batches of eggs with a fertilization rate of at least 90% were collected 30 minutes after spawning and rinsed several times in Dutch Standard Water (DSW; demineralized water supplemented with NaHCO3 (100 mg/l), KHCO3 (20 mg/l), CaCl2 ·2H2O (200 mg/l), and MgSO4·7H2O (180 mg/l) and then aerated for 24h at 27°C) to remove any coagulated eggs or debris before exposure. After rinsing, the eggs were evenly distributed among the test concentrations. Hereafter, embryos within the 4- to 64-cell stage were selected and transferred to a 24-well plate containing 2 ml of test medium per well. One embryo was transferred to one well and all necessary controls were included. Embryos were kept in an incubator at 26.5°C ± 1°C with a photoperiod of 14h light: 10h dark.

At 72 hours post fertilization (hpf) the embryos were evaluated under a Leica Labovert FS microscope using the general morphology score (GMS) system to quantify developmental retardation and teratogenicity. For microarray analyses embryos were collected at 24 hpf.

### Example 3: Test compounds and concentrations (BMCGMS20)

Embryos were exposed to equipotent concentrations of the test compounds. In our previous study, methoxyacetic acid (MAA, cat. no. 194557), ethoxyacetic acid (EAA, cat. no. 137111), phenoxyacetic acid (PAA, cat. no. 77740, negative control), flusilazole (FLU, cat. no. 45753) and cyproconazole (CYP, mixture of diastereomers, cat. no. 46068, all from Sigma) were all tested in a concentration range in the ZET in three independent runs to derive concentration-response curves for benchmark concentration calculation [11]. A representative concentration-response curve using the exponential model with the lowest number of parameters which gave the best fit was selected to calculate the benchmark concentration for the GMS (BMCGMS). A decrease in GMS of 20% was chosen as the benchmark response (BMR) for calculating the corresponding BMCGMS20. This is equal to a GMS of 12 points at 72 hpf in exposed compared to the maximum 15 points in normally developed embryos. All calculations were done using PROAST software.

MAA and EAA were tested at the equimolar concentrations because their concentration-response curves were very similar and their confidence intervals of the BMCGMS20 overlapped. PAA was included as a negative control and showed no effects, for that reason no BMC GMS20 was calculated. PAA was tested at an equimolar concentration as MAA and EAA. Both triazoles were tested at their BMC GMS20. The pH of all test media ranged from or was adjusted to 7.4-8.4, and 02-concentration was at least 6.5 mg/l before and after the test. All compounds were diluted in DSW with a final DMSO concentration of 0.2% vol/vol. DMSO 0.2% vol/vol was also included as control.

### Example 4: RNA isolation and processing

For microarray analysis, 20 zebrafish embryos were exposed to BMCGMS20 of MAA, EAA, PAA, FLU and CYP. At 24 hpf 20 embryos per sample were collected and stored in RNAlater to stabilize RNA. For MAA, PAA, FLU and controls 8 biological samples were exposed and used for transcriptomics and for EAA and CYP 7 biological samples. In parallel, 48 embryos per compound and control were exposed and morphologically evaluated at 72 hpf as described before to assess if the concentration tested induced the 20% decrease in GMS. Total RNA was isolated from the embryos using the RNeasy mini kit with an additional DNase treatment (RNase free DNase set) according to the manufacturer's instructions (all from Qiagen, Venlo, The Netherlands). RNA concentration was measured on the NanoDrop spectrophotometer (ND-1000, NanoDrop Technologies Inc., Wilmington, DE) and RNA integrity was assessed by automated gel electrophoresis using the RNA 6000 Nano Chip kit (Bioanalyzer 2100, Agilent technologies, Amstelveen, The Netherlands). Total RNA samples with an RNA Integrity Number (RIN)>7 were used for further analysis.

### Example 5: expression profiling

Technical handling of the microarrays was performed at the MicroArray Department (MAD) of the University of Amsterdam, The Netherlands. Per sample, 500 ng total RNA was combined with Spike A and amplified and labeled according to the Agilent Two-Color Microarray-Based Gene Expression Analysis guide version 5.5 (G4140-90050, Agilent technologies, Amstelveen, The Netherlands). For the common reference an equimolar pool of all test samples was made and 500 ng samples were amplified similarly as the test samples with the exception that Spike B was used. Synthesized cRNA was purified with the RNeasy MinElute Cleanup Kit (Qiagen, Venlo, The Netherlands). Yields of cRNA and CyDye incorporation were measured on the NanoDrop spectrophotometer and the quality was inspected with the RNA 6000 Nano Chip kit on the Bioanalyzer. Each hybridization mixture was made from 825 ng Test (Cy3) and 825 ng Reference (Cy5) material according to the Agilent protocol (Agilent Technologies). The samples were loaded onto 4x44k D. rerio microarrays (Design ID:022562, Agilent Technologies) and hybridized for 17 hours at 65°C. Afterwards the slides were washed and scanned (16 bit, 5 µm resolution) in an ozone-free room with the Agilent G2505C scanner as described in the manual. Data was extracted with Feature Extraction (v10.7.3.1, Agilent Technologies) with the GE2_107_Sep09 protocol for two-color Agilent microarrays.

### Example 6: Data analysis

Quality control was performed on raw microarray data using a scatterplot and MA-plot as well as a normal probability plot to assess signal distribution. The positive and negative controls on the microarray slides were solely used for quality control and therefore excluded from further analysis.

Microarray data was normalized and further analyzed using R statistical software. Normalization of the raw microarray data was done in a three step approach as described by Janssen *et al* [28]. A t-test was performed between a compound exposed sample and the control sample. Any of the four genes described herein showed a significant (p<0.001) and at least 50% change in expression level as compared to the reference value. These genes were either up-or down-regulated when exposed to developmentally toxic compounds compared to an unexposed reference value.

### Example 7: testing of 4 developmentally toxic compounds and one non-developmental toxic compound

As described in the previous examples embryos were exposed to PAA, MAA, EAA, FLU, CYP and the control DMSO. At 24 hpf microarray analysis was performed. T-test between the compound exposed sample and control sample revealed 4 genes that were regulated by all of the embryotoxic compounds MAA, EAA, FLU and CYP with a p-value <0.001 and a fold ratio (FR) >1.5 (either up- or downregulated), with an additional criterium that genes were not significant for PAA and had a FR<1.2 (up- or downregulated).

### References

[1] Genschow E, Spielmann H, Scholz G, Pohl I, Seiler A, Clemann N, et al. Validation of the embryonic stem cell test in the international ECVAM validation study on three in vitro embryotoxicity tests. Altern Lab Anim. 2004;32:209-44.
[2] Nagel R. DarT: The embryo test with the Zebrafish Danio rerio--a general model in ecotoxicology and toxicology. Altex. 2002;19 Suppl 1:38-48.
[3] Piersma AH, Genschow E, Verhoef A, Spanjersberg MQ, Brown NA, Brady M, et al. Validation of the postimplantation rat whole-embryo culture test in the international ECVAM validation study on three in vitro embryotoxicity tests. Altern Lab Anim. 2004;32:275-307.
[4] Spielmann H, Seiler A, Bremer S, Hareng L, Hartung T, Ahr H, et al. The practical application of three validated in vitro embryotoxicity tests. The report and recommendations of an ECVAM/ZEBET workshop (ECVAM workshop 57). Altern Lab Anim. 2006;34:527-38.
[5] Kimmel CB, Ballard WW, Kimmel SR, Ullmann B, Schilling TF. Stages of embryonic development of the zebrafish. Dev Dyn. 1995;203:253-310.
[6] Yang L, Ho NY, Alshut R, Legradi J, Weiss C, Reischl M, et al. Zebrafish embryos as models for embryotoxic and teratological effects of chemicals. Reproductive Toxicology. 2009;28:245-53.
[7] Hill AJ, Teraoka H, Heideman W, Peterson RE. Zebrafish as a model vertebrate for investigating chemical toxicity. Toxicol Sci. 2005;86:6-19.
[8] Fleming A. Zebrafish as an alternative model organism for disease modelling and drug discovery: implications for the 3Rs. NC3Rs. 2007:1-7.
[9] European Commission. Council Directive 86/609/EEC of 24 November 1986 on the approximation of laws, regulations and administrative provisions of the Member States regarding the protection of animals used for experimental and other scientific purposes. Official Journal L 358 , 18/12/1986. 1986: P. 0001 - 28.
[10] Brannen KC, Panzica-Kelly JM, Danberry TL, Augustine-Rauch KA. Development of a zebrafish embryo teratogenicity assay and quantitative prediction model. Birth Defects Res B Dev Reprod Toxicol. 2010;89:66-77.
[11] Hermsen SAB, van den Brandhof E-J, van der Ven LTM, Piersma AH. Relative embryotoxicity of two classes of chemicals in a modified zebrafish embryotoxicity test and comparison with their in vivo potencies. Toxicology in Vitro. 2011;25:745-53.
[12] de Jong M, Rauwerda H, Bruning O, Verkooijen J, Spaink H, Breit T. RNA isolation method for single embryo transcriptome analysis in zebrafish. BMC Research Notes. 2010;3:73.
[13] Brown NA, Holt D, Webb M. The teratogenicity of methoxyacetic acid in the rat. Toxicol Lett. 1984;22:93-100.
[14] Hanley TR, Yano BL, Nitschke KD, John JA. Comparison of the teratogenic potential of inhaled ethylene glycol monomethyl ether in rats, mice, and rabbits. Toxicology and Applied Pharmacology. 1984;75:409-22.
[15] Nagano K, Nakayama E, Oobayashi H, Yamada T, Adachi H, Nishizawa T, et al. Embryotoxic effects of ethylene glycol monomethyl ether in mice. Toxicology. 1981;20:335-43.
[16] Bagchi G, Hurst CH, Waxman DJ. Interactions of methoxyacetic acid with androgen receptor. Toxicology and Applied Pharmacology. 2009;238:101-10.
[17] Henley DV, Korach KS. Endocrine-disrupting chemicals use distinct mechanisms of action to modulate endocrine system function. Endocrinology. 2006;147:S25-32.
[18] Di Renzo F, Broccia ML, Giavini E, Menegola E. Stage-dependent abnormalities induced by the fungicide triadimefon in the mouse. Reprod Toxicol. 2010.
[19] Farag AT, Ibrahim HH. Developmental toxic effects of antifungal flusilazole administered by gavage to mice. Birth Defects Res B Dev Reprod Toxicol. 2007;80:12-7.
[20] Machera K. Developmental toxicity of cyproconazole, an inhibitor of fungal ergosterol biosynthesis, in the rat. Bulletin of Environmental Contamination and Toxicology. 1995;54:363-9.
[21] Marotta F, Tiboni GM. Molecular aspects of azoles-induced teratogenesis. Expert Opin Drug Metab Toxicol. 2010;6:461-82.
[22] Menegola E, Broccia ML, Di Renzo F, Giavini E. Postulated pathogenic pathway in triazole fungicide induced dysmorphogenic effects. Reproductive Toxicology. 2006;22:186-95.
[23] Papis E, Bernardini G, Gornati R, Menegola E, Prati M. Gene expression in Xenopus laevis embryos after Triadimefon exposure. Gene Expression Patterns. 2007;7:137-42.
[24] Tiboni GM, Marotta F, Carletti E. Fluconazole alters CYP26 gene expression in mouse embryos. Reproductive Toxicology. 2009;27:199-202.
[25] Groppelli S, Pennati R, De Bernardi F, Menegola E, Giavini E, Sotgia C. Teratogenic effects of two antifungal triazoles, triadimefon and triadimenol, on Xenopus laevis development: Craniofacial defects. Aquatic Toxicology. 2005;73:370-81.
[26] Menegola E, Broccia ML, Di Renzo F, Giavini E. Antifungal triazoles induce malformations in vitro. Reproductive Toxicology. 2001;15:421-7.
[27] Menegola E, Broccia ML, Di Renzo F, Massa V, Giavini E. Craniofacial and axial skeletal defects induced by the fungicide triadimefon in the mouse. Birth Defects Res B Dev Reprod Toxicol. 2005;74:185-95.
[28] Janssen R, Pennings J, Hodemaekers H, Buisman A, van Oosten M, de Rond L, et al. Host transcription profiles upon primary respiratory syncytial virus infection. J Virol. 2007;81:5958-67.

## Claims

1. Method for determining the developmental toxicity of a compound comprising the steps of
a) Providing a 2 - 128 cell stage fish embryo,
b) Contacting the fish embryo with the compound,
c) Allowing the embryo to develop for an amount of time in the presence of the compound,
d) Isolating total RNA from the embryo,
e) Determining the expression level of at least one gene selected from the group consisting of *cyp26a1, si:rp71-39b20.7, vmhc* and *hspb12,*
f) Comparing the expression level of said at least one gene with a predetermined reference value,
wherein an increase or decrease of the expression level of said gene indicates that the compound is developmentally toxic.

2. Method according to claim 1 wherein the increase or decrease of the expression level of said gene is at least 50% of the reference value.

3. Method according to claims 1 or 2 wherein the fish embryo is provided in a 4 - 64 cell stage.

4. Method according to claims 1 - 3 wherein the fish is a zebrafish

5. Method according to claims 1 - 4 wherein the amount of time is between 12 and 72 hours.

6. Method according to claim 5 wherein the amount of time is between 20 and 28 hours.

7. Method according to claim 1 wherein the subsequent method steps a) to f) are repeated and wherein the increase or decrease of said gene expression is an average of the independent repeats.

8. Method according to claims 1 - 7 wherein the compound is contacted with the embryo in a concentration just below its toxic level.

9. Method according to claims 1 - 8 wherein the at least one gene is two genes, three genes or four genes.
